# EUROPÄISCHE PATENTSCHRIFT

(11) **EP 2 630 982 B1**
(45) Veröffentlichungstag und Bekanntmachung des Hinweises auf die Patenterteilung: **05.04.2017**
(21) Anmeldenummer: 12156555.0
(22) Anmeldetag: 22.02.2012
(51) Int. Cl.: A61B 18/14, A62B 18/02, A61M 39/10, A61M 39/16, A61M 39/20, A61B 18/02

(54) **Kryochirurgisches Instrument und Stecker mit Entlüftungsöffnung für dieses**
Surgical cryoprobe instrument and vented connector for same
INSTRUMENT CRYOCHIRURGICAL et fiche ventilée pour celui-ci

(43) Veröffentlichungstag der Anmeldung: 28.08.2013
(73) Patentinhaber: Erbe Elektromedizin GmbH, 72072 Tübingen (DE)
(72) Erfinder: Amann, Markus, 72070 Tübingen (DE); Besch, Hansjörg, 72810 Gomaringen (DE); Kühner, Ralf, 70567 Stuttgart (DE)
(74) Vertreter: Rüger, Barthelt & Abel

(56) Entgegenhaltungen:
- WO-A1-99/65410
- WO-A1-2007/073810
- GB-A- 1 536 682
- US-A- 3 971 383
- US-A- 5 520 682
- US-A- 5 573 532
- US-A1- 2002 089 177
- US-A1- 2003 028 182
- US-A1- 2004 078 033

## Beschreibung

Die Erfindung betrifft allgemein ein medizinisches Instrument und insbesondere dessen Anschlussstecker.

Es sind aus der Praxis medizinische Instrumente bekannt, die einen Instrumentenkopf aufweisen, über den ein Operateur oder ein sonstiger medizinischer Behandler an einem menschlichen oder tierischen Patienten physikalische Wirkungen erreicht, bei denen beispielsweise Gewebe verändert, getrennt oder abgetragen wird.

Aus der US-A 5,520,682 ist ein kryochirurgisches Instrument bekannt, das zwei Anschlüsse zum Zu- und Ableiten von Kryofluid aufweist. Die zuführende Leitung ist dabei koaxial innerhalb der abführenden Leitung angeordnet. Die zuführende Leitung und die abführende Leitung erstrecken sich in dieser Koaxialanordnung durch ein-Griffstück. Am proximalen Ende desselben führen sie parallel zueinander aus dem Griffstück und können dort über Schlauchleitungen mit einer entsprechenden speisenden Einrichtung verbunden werden.

Zur thermischen Isolation ist das Gehäuse der Anordnung bis kurz vor die distale Spitze evakuierbar. Der Zwischenraum zwischen der abführenden Leitung und dem Gehäuse ist dazu mit einem Pumpstutzen versehen, über den das Instrument an eine Vakuumeinrichtung angeschlossen werden kann. Nach Evakuierung des wärmeisolierenden Vakuumraums kann der Pumpstutzen verschlossen werden.

Es sind Instrumente bekannt, bei denen dazu der vorhandene Instrumentenkopf mit einem Fluid, bspw. einem Kryofluid, versorgt werden muss, um die gewünschte physikalische Wirkung zu erbringen. Dazu ist der Instrumentenkopf mit einer Versorgungsleitung versehen, die den Instrumentenkopf mit einem Instrumentenstecker verbindet. Letzterer ist an ein medizinisches Gerät angepasst, welches das Instrument mit den gewünschten Medien, insbesondere Fluiden, sowie elektrischen Parametern versorgt. Der Instrumentenstecker weist dazu mindestens einen Fluidstecker auf, der mit einer entsprechenden Buchse des Geräts in Verbindung bringbar ist. Außerdem ist an dem üblicherweise im Wesentlichen rotationssymmetrisch ausgebildeten Stecker eine seitliche Belüftungsöffnung angebracht, aus der bspw. durch Undichtigkeiten innerhalb der Versorgungsleitung frei gewordene Fluide gefahrlos ausgeleitet werden können. So werden unerwünschte Wirkungen vermieden, wie bspw. das Aufblähen einer Schutzhülle der Versorgungsleitung.

Der Instrumentenstecker muss vielfältigen Anforderungen genügen. Beispielsweise stehen die dem Instrumentenkopf zuzuleitenden Fluide häufig unter beträchtlichem Druck, so dass diese Fluide keineswegs unkontrolliert entkommen dürfen. Auch kann eine plötzliche Entspannung der verwendeten Fluide zu extremer Kälteentwicklung und somit der Gefahr von Verletzungen führen. Herkömmliche Instrumentenstecker wurden deshalb meist aus robusten, kompliziert geformten Drehteilen gefertigt, was entsprechend aufwändig ist.

Weiter muss, zumindest bei mehrfach zu verwendenden Instrumenten, die Sterilisierung der Instrumente in Betracht gezogen werden. Die Sterilisierung bezieht sich hierbei auf alle von außen zugänglichen Flächen. Es muss aber verhindert werden, dass Waschflüssigkeit, Spülflüssigkeit oder dergleichen, in die Fluidkanäle des Instruments eindringt, weil Reste dieser Wasch- oder Spülflüssigkeit, wenn sie im Instrument verbleiben, beim späteren Einsatz zu erheblichen Störungen an dem Instrument und letztlich zu Schäden am Patienten führen können.

Daraus ergibt sich die der Erfindung zugrunde liegende Aufgabe, ein verbessertes, kostengünstiges Instrument zu schaffen, das einerseits einen einfachen und übersichtlichen Aufbau aufweist, der andererseits leicht zu reinigen, insbesondere sterilisierbar ist.

Diese Aufgabe wird mit dem Instrument nach Anspruch 1 gelöst:
Das erfindungsgemäße Instrument weist einen Instrumentenkopf bestehend aus einem Handgriff und einem Anwendungsteil auf, der wenigstens ein Wirkteil aufweist. Dieser Instrumentenkopf wird über eine Versorgungsleitung mit mindestens einem Fluid in gasförmigem, flüssigem oder superkritischem Aggregatzustand versorgt. Das Fluid kann bspw. Kohlendioxyd, Stickstoffmonoxid, Argon, Stickstoff, auch in flüssiger Form, oder ein sonstiges Fluid oder Fluidgemisch sein. Dieses Fluid wird dem Instrumentenkopf durch eine Versorgungsleitung zugeführt. Gegebenenfalls ist eine weitere Versorgungsleitung vorgesehen, die das Fluid aus dem Instrumentenkopf zurück zu dem Instrumentenstecker führt. An dem Instrumentenstecker sind je nach Zahl der Versorgungsleitungen zwei oder mehrere Fluidstecker angeordnet, die dazu eingerichtet sind, mit entsprechenden Buchsen zusammenzuwirken, die an einem medizinischen Gerät zur Versorgung des Instruments vorgesehen sind.

Der Instrumentenstecker weist eine flache Stirnseite auf, an welcher mindestens zwei Fluidstecker angeordnet sind. Des Weiteren ist an der gleichen flachen Stirnseite wenigstens eine Entlüftungsöffnung vorgesehen, die den Innenraum des Mantels der Versorgungsleitung mit der Umwelt verbindet. Der Mantel hüllt die mindestens eine Fluidleitung ein.

Wird eine der Fluidleitungen an irgendeiner Stelle entlang ihrer Länge undicht oder entkommt Fluid in dem Instrumentenkopf aus dem geschlossenen Kreislauf der Fluidleitungen, gelangt dieses Fluid zunächst in den Innenraum des Mantels, der ebenso wie der Stecker selbst und wie der Instrumentenkopf vorzugsweise hermetisch gegen die Umgebung abgeschlossen ist. Jedoch ist dieser Innenraum an der Stirnseite des Steckers belüftet, so dass sich in dem Mantel kein Druck aufbauen kann. Freigewordenes Fluid strömt oder fließt an der Stirnseite des Instrumentensteckers frei ab. Weitergehende Schädigungen des Instruments sowie Schädigungen des Patienten und/oder des Operateurs werden dadurch vermieden.

Die Anordnung sowohl des mindestens eines Instrumentsteckers wie auch der Belüftungsöffnung an der Stirnseite des Steckers hat erhebliche Auswirkung auf die Sterilisierbarkeit des Instruments einschließlich seines Steckers. Eine einfache an die Stirnfläche anzusetzende Abdeckkappe kann sowohl den oder die Fluidstecker als auch die Entlüftungsöffnung schließen, so dass keine Feuchtigkeit beispielsweise in Form von Waschfluid, Spülfluid, Seife oder dergleichen, in die inneren Kanäle, d.h. in die Fluidleitung oder den Innenraum des Mantels eindringen kann. Gerade Letzteres könnte besonders verderblich wirken. Wenn bspw. Reste von Wasch- oder Spülfluid nach und nach die Entlüftungsöffnung zusetzen, wäre deren schützende Wirkung im Fall eines Bruchs oder einer sonstigen Undichtigkeit einer Fluidleitung nicht mehr gegeben. Die Erfindung verhindert dies.

Außerdem ermöglicht die Anordnung mehrerer Fluidstecker an ein und derselben Stirnseite einen einfachen Steckeraufbau und die Verwendung einer großen Anzahl von Gleichteilen. Der Fluidstecker für die Fluidzuführung und mindestens ein weiterer Fluidstecker für die Fluidabführung können einheitlich aufgebaut sein. Die Fluidstecker sind vorzugsweise parallel zueinander orientiert. Die Entlüftungsöffnung kann zwischen den Fluidsteckern angeordnet sein. Sie kann mit diesen auf einer gemeinsamen Linie oder auch von einer gemeinsamen Linie abweichend angeordnet sein. Diese Steckeranordnung kann sich sowohl für Instrumente mit wenigstens abschnittsweise starrem Anwendungsteil wie auch für Instrumente mit flexiblem Anwendungsteil eignen.

Der Instrumentenstecker ist vorzugsweise als Flachstecker ausgebildet. Unabhängig davon, umfasst er vorzugsweise zwei Gehäuseschalen, die an einer Fuge aneinander anschließen. Vorzugsweise ist dabei die Stirnseite fugenlos ausgebildet. Dies ermöglicht eine besonders einfache Steckermontage. Der von den Gehäuseschalen umschlossene Innenraum ist vorzugsweise vollständig von einer Vergussmasse gefüllt. Damit werden einerseits die in dem Stecker vorhandenen Leitungsanschlüsse fixiert und gesichert und andererseits Toträume, die zu sterilisieren wären, vermieden.

Dem Instrumentenstecker ist vorzugsweise eine Abdeckkappe zugeordnet, mit deren Hilfe sowohl die Fluidstecker wie auch die Entlüftungsöffnung so verschlossen werden können, dass beim Sterilisieren keine Flüssigkeiten in die inneren Kanäle, insbesondere die Fluidleitungen und den Innenraum des Mantels, eindringen und dort verbleiben.

Weitere Einzelheiten vorteilhafter Ausführungsformen der Erfindung sind Gegenstand der nachfolgenden Beschreibung oder von Unteransprüchen. Es zeigen:
Figur 1 ein erfindungsgemäßes Instrument, in schematisierter Perspektivdarstellung,
Figur 2 den Instrumentenstecker des Instruments nach Figur 1, im Horizontalschnitt,
Figur 3 die Versorgungsleitung des Instruments nach Figur 1, im Querschnitt,
Figur 4 den Instrumentenstecker nach Figur 1, in einer schematisierten Perspektivdarstellung,
Figur 5 den Instrumentenstecker und seine vertikal geschnittene Abschlusskappe, in Prinzipdarstellung, und
Figur 6 den Instrumentenstecker und die Abdeckkappe, in Draufsicht.

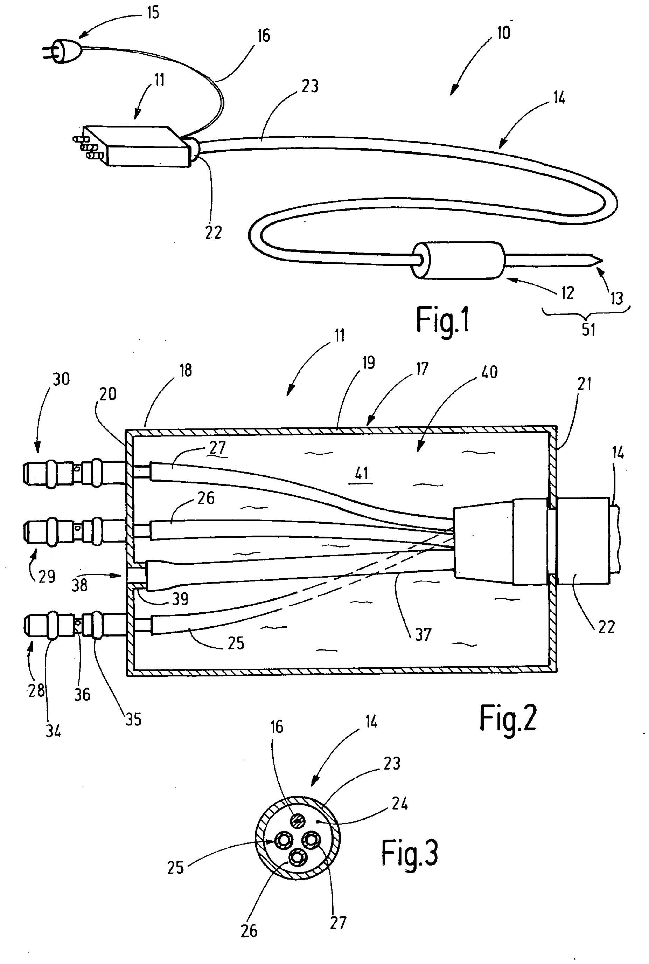

In Fig. 1 ist ein medizinisches, insbesondere ein chirurgisches Instrument 10 veranschaulicht, das zum Anschluss an ein nicht weiter veranschaulichtes medizinisches Gerät vorgesehen ist. Das Instrument 10 umfasst als wesentliche Bestandteile einen Instrumentenstecker 11, ein Anwendungsteil 51, das einen Instrumentenkopf 12, mit einem hier allgemein als Werkzeug 13 bezeichneten Wirkteil umfasst, sowie eine Versorgungsleitung 14, die das Anwendungsteil 51 mit dem Instrumentenstecker 11 verbindet. Im vorliegenden Beispiel ist das Werkzeug 13 ein kryochirurgisches Werkzeug, das an mindestens einer Stelle, beispielsweise seiner Spitze, die gewünschte Wirkung, beispielsweise eine Kältewirkung, entfalten kann. Das dazu erforderliche Kryofluid kann über den Instrumentenstecker 11 zugeführt werden. Optional können weitere oder andere Medien, beispielsweise elektrischer Strom, z.B. HF-Strom oder dergleichen, zugeführt werden. Dazu kann ein weiterer Stecker 15 vorgesehen sein, der mit dem Instrumentenstecker 11 über eine Leitung 16 verbunden ist, die sich durch die Versorgungsleitung 14 fortsetzt und zu dem Instrumentenkopf 12 führt.

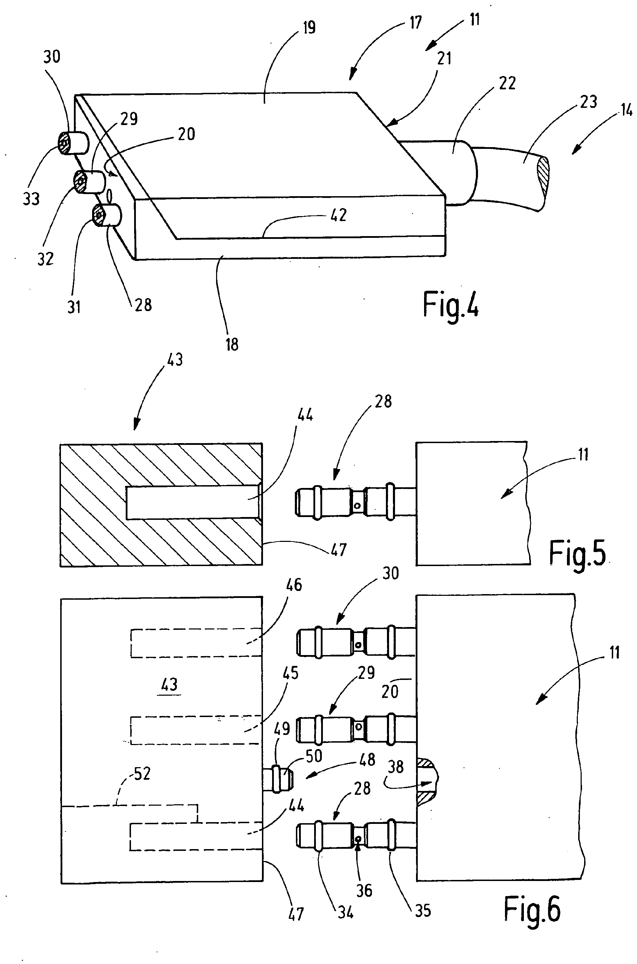

Fig. 2 zeigt den inneren Aufbau des Instrumentensteckers 11 schematisch. Der Instrumentenstecker 11 weist ein Gehäuse 17 auf, das vorzugsweise aus mehreren Gehäuseteilen, beispielsweise zwei Gehäuseschalen 18, 19 besteht, wie sie aus Fig. 4 ersichtlich sind. Z.B. ist das Gehäuse ein Flachgehäuse, das im Wesentlichen quaderförmig ausgebildet ist oder eine von der Quaderform abgeleitete gefälligere Gehäuseform hat. Das Gehäuse 17 weist eine vorzugsweise ebene vordere Stirnseite 20 auf, von der sich Seitenflächen des Gehäuses weg erstrecken, die zu der Stirnseite 20 z.B. im Wesentlichen rechtwinklig orientiert sind. Die Seitenflächen können flach, dabei auch leicht gewölbt und mit sonstigen Strukturen versehen sein, z.B. Rippen, Noppen oder dergleichen.

An die der Stirnseite 20 gegenüberliegende Rückseite 21 des Gehäuses 17 schließt die Versorgungsleitung 14 an. Zu dieser gehört im unmittelbaren Anschluss an den Instrumentenstecker 11 eine Fassung 22, beispielsweise in Gestalt einer Gummitülle oder dergleichen. Die Fassung 22 schließt vorzugsweise hermetisch dicht an die Außenumfangsfläche eines äußeren Mantels 23 der Versorgungsleitung 14 an. Der Mantel 23 ist vorzugsweise als flexibler Schlauch ausgebildet. Er kann, falls gewünscht, steife Abschnitte aufweisen oder auch auf gesamter Länge flexibel sein. Er kann ein- oder mehrteilig ausgebildet sein.

Der Mantel 23 umschließt, wie aus Fig. 3 hervorgeht, einen Innenraum 24, der sich unterbrechungsfrei längs durch den gesamten den Mantel 23 bildenden Schlauch von der Fassung 22 zu dem Anwendungsteil 51 bzw. dem Instrumentenkopf 12 erstreckt und mit dessen Innenraum, falls vorhanden, kommuniziert. In dem Innenraum 24 sind eine oder mehrere Fluidleitungen 25, 26, 27 angeordnet, die vorzugsweise als flexible Schläuche ausgebildet sind. Außerdem kann sich, wie oben erwähnt, die Leitung 16 durch die Versorgungsleitung 14 und somit durch den Innenraum 24 erstrecken. Wie in Fig. 3 dargestellt, füllen die gegebenenfalls vorhandene Leitung 16, sowie die Versorgungsleitungen 25-27 den Innenraum 24 nicht vollständig aus. Vielmehr verbleibt ein gewisser freier Strömungsquerschnitt der allerdings wesentlich geringer sein kann, als in Fig. 3 dargestellt.

Die Fluidleitungen 25, 26, 27 erstrecken sich durch die Fassung 22 hindurch bis zu der gegenüberliegenden Gehäuseseite, wo sie an Fluidstecker 28-30 angeschlossen sind. Die Fluidstecker 28-30 sind an der Stirnseite 20 angeordnet und erstrecken sich vorzugsweise im rechten Winkel zu dieser und parallel zueinander von der Stirnseite 20 weg. Sie weisen jeweils einen inneren Kanal 31-33 auf, wie es aus Fig. 4 hervorgeht. Diese Kanäle 31-33 kommunizieren mit den inneren Kanälen der Fluidleitungen 25-27. Die Fluidstecker 28-30 sind jeweils stirnseitig geschlossen und ansonsten im Wesentlichen zylindrisch ausgebildet. Sie sind außerdem untereinander vorzugsweise vollständig gleich ausgebildet. Die Beschreibung des Fluidsteckers 28 gilt entsprechend für die Fluidstecker 29 und 30.

Der Fluidstecker 28 weist zwei im axialen Abstand voneinander angeordnete Dichtungen, beispielsweise O-Ringe 34, 35 auf, die in entsprechenden O-Ringnuten sitzen. Zwischen diesen kommuniziert der innere Kanal 31 mit mindestens einer Radialbohrung 36, die in einer etwas vertieften Radialnut angebracht sein kann. Vorzugsweise weisen die beiden O-Ringe 34, 35 denselben Außendurchmesser auf, der auf den Innendurchmesser der Bohrung 44 abgestimmt ist. Ist der Fluidstecker 28 in der Bohrung 44 eingeführt und wird über die Fluidleitung 52 unter Druck stehendes Fluid zugeführt, entsteht aufgrund der gleichen Außendurchmesser der O-Ringe 34, 35 eine druckunterstütze Hemmung des Fluidsteckers 28 in der Bohrung 44. Unterschiedliche Außendurchmesser der O-Ringe können die Wirkverbindung des Fluidsteckers 28 mit der Bohrung 44 beeinflussen.

Der Innenraum 24 der Versorgungsleitung 14 endet in oder an der Fassung 22. Ein Entlüftungsschlauch 37 oder ein entsprechendes Rohr verbindet den Innenraum 24 mit einer an der Stirnseite 20 angebrachten Entlüftungsöffnung 38 beispielweise in Form einer Entlüftungsbohrung 38, die der Druckentlastung des Innenraums 24 dient und über die der Innenraum 24 direkt oder mittels einer Verbindung mit dem Gerät mit der Atmosphäre in Verbindung steht. An der Entlüftungsbohrung 38 kann ein an der Stirnseite 20 ausgebildeter nach innen ragender Fortsatz 39 vorgesehen sein, auf dem der Entlüftungsschlauch 37 sitzt.

Das Gehäuse 17 umschließt einen Innenraum 40, der vorzugsweise vollständig mit Vergussmasse 41 gefüllt ist. Die Vergussmasse 41 umhüllt dabei die Fluidleitungen 25-27, sowie den Entlüftungsschlauch 37 und den in dem Gehäuse 17 befindlichen Teil der Fassung 22. Diese wird dadurch fixiert und zu dem Innenraum 40 hin abgedichtet. Auch eine zwischen den Gehäuseschalen 18, 19 vorhandene Gehäusefuge 42 wird durch die Vergussmasse 41 abgedichtet. Vorzugsweise ist die Gehäusefuge 42 außerhalb der Stirnseite 20 angeordnet, so dass letztere unterbrechungsfrei ist.

Das insoweit beschriebene Instrument 10 kann beispielsweise als Einweg-Instrument vorgesehen sein. Im Einsatz wird sein Instrumentenstecker 11 an einem entsprechenden Steckplatz eines zur Versorgung vorgesehenen Geräts gesichert. Dabei treten die Fluidstecker 28, 29, 30 in entsprechende Steckbuchsen ein. Beispielsweise empfängt der Fluidstecker 29 über die ihm zugeordnete Steckbuchse ein geeignetes Fluid, beispielsweise flüssigen Stickstoff, der z.B. durch die Fluidleitung 26 durch die Versorgungsleitung 14 hindurch zu dem Instrumentenkopf 12 geleitet wird. Über mindestens eine der beiden Fluidleitungen 25 und/oder 27 gelangt verbrauchtes Fluid, beispielsweise verdampfter und somit gasförmiger Stickstoff, zu den Fluidsteckern 28 und/oder 30 zurück und wird von dem Gerät wieder aufgenommen. Der dem Instrumentenkopf 12 und insbesondere dem Werkzeug 13 zugeleitete flüssige Stickstoff kann beispielsweise für Kryoanwendungen in dem Werkzeug 13 verdampfen und dabei Wärme aufnehmen, die mit dem gasförmigen Stickstoff über die Fluidleitungen 25, 27 abtransportiert wird. Dabei verlässt das entsprechende Fluid, hier beispielsweise Stickstoff, den von den Fluidleitungen 25-27 und den Instrumentenkopf 12 gebildeten Kreislauf nicht.

Sollte allerdings an irgendeiner Stelle eine Leckage auftreten, kann das verwendete Fluid, beispielsweise flüssiger oder gasförmiger Stickstoff, in den Innenraum 24 der Versorgungsleitung 14 gelangen. Selbst wenn flüssiger Stickstoff nur in sehr geringen Mengen in diesen Innenraum 24 gelangt, würde er diesen, wäre er nach außen abgeschlossen, infolge der beim Verdampfen auftretenden Volumenzunahme sofort stark aufblähen. Gleiches wäre zu verzeichnen, wenn unter hohem Druck stehende Fluide durch die Fluidleitungen 25, 26 und/oder 27 geleitet würden. Im vorliegenden Fall können solche Leckfluide jedoch keinen Druckstau verursachen. Sie werden vielmehr über den Entlüftungsschlauch 37 und die Entlüftungsbohrung 38 nach außen abgeleitet. An dem geräteseitigen Steckplatz kann dazu eine entsprechend mit der Entlüftungsbohrung 38 fluchtende weitere Öffnung vorgesehen sein.

Das insoweit beschriebene Instrument 11 kann insbesondere auch als mehrfach verwendbares, sterilisierbares Instrument ausgebildet sein. In diesem Fall kann ihm eine Abdeckkappe 43 zugeordnet sein, wie sie aus den Figuren 5 und 6 ersichtlich ist. Diese weist für jeden Fluidstecker 28 bis 30 jeweils eine Bohrung 44, 45, 46 auf, deren Positionen den Positionen der Fluidstecker 28 bis 30 entsprechen. Die Bohrungen 44 bis 46 weisen vorzugsweise zylindrische geschlossene Wandungen auf, an denen die jeweiligen Dichtungen 34, 35 abdichtend anliegen. Auf diese Weise werden die Radialbohrungen 36 und somit die Kanäle 31 bis 33 abgedichtet. Die Bohrungen 44 bis 46 erstrecken sich vorzugsweise von einer flachen Stirnseite 47 der Abdeckkappe 43 parallel zueinander in den Körper der Abdeckkappe 43 hinein.

An der Stirnseite 47 kann außerdem ein geeignetes Dichtungsmittel 48 für die Entlüftungsbohrung 38 vorgesehen sein, wie insbesondere aus Figur 6 hervorgeht. Beispielsweise kann dieses Dichtungsmittel 48 ein mit einer Dichtung 49 versehener Zapfen 50 sein. Dieser kann in die Entlüftungsbohrung 38 hinein passen, so dass die Dichtung 49 an der inneren Wandung der Entlüftungsbohrung 38 abdichtend anliegt, wenn die Stirnseite 47 der Abdeckkappe 43 an der Stirnseite des Instrumentensteckers 11 anliegt.

Zum Sterilisieren des Instruments 10 wird die Abdeckkappe 43 so mit dem Instrumentenstecker 11 verbunden, dass alle Fluidstecker 28 bis 30 in den Bohrungen 44 bis 46 sitzen und dass die Entlüftungsbohrung 38 durch das Dichtungsmittel 48 verschlossen ist. Zur sicheren Verbindung der Abdeckkappe 43 mit dem Instrumentenstecker 11 können die Abdeckkappe 43 und der Instrumentenstecker 11 zueinander komplementäre Verbindungsmittel (nicht dargestellt) beispielsweise in Form von Nut und Feder oder Haken und Öse oder dergleichen aufweisen. Es kann nun eine Sterilisierung durch Waschen, Spülen und das Einwirken von sonstigen Fluiden wie bspw. Dampf vorgenommen werden. Ein Eindringen von Waschmitteln oder anderen Substanzen in das Innere der Fluidleitungen 25 bis 27 und insbesondere in den Innenraum 24 ist ausgeschlossen.

Der Instrumentenstecker 11 gemäß Figur 4 kann mit verschieden ausgebildeten chirurgischen Instrumenten verbunden sein. Dabei unterscheidet man zwischen so genannten starren Sonden und flexiblen Sonden. Eine starre Sonde weist einen starren druckfesten Anwendungsteil 51 auf, der beispielsweise für Drücke bis zu 65 bar und mehr ausgelegt ist. Um diese Druckfestigkeit zu gewährleisten, müssen alle fluidführenden Teile sowie alle Verbindungsmittel eines Anwendungsbereichs 51, wie beispielsweise ein Kapillarrohr, eine Verschlusskappe, einen Flansch, eine Mutter und Dichtmittel in Form von O-Ringen, druckfest ausgebildet sein. Zurzeit ist es technisch noch sehr schwierig, in den erforderlichen geringen Abmessungen, diese fluidführenden Teile sowie die Verbindungsmittel druckfest und flexibel herzustellen. Um zu verhindern, dass eine flexible Sonde mit einem Anwendungsteil 51 das nicht druckfest ausgelegt ist, mit einem zu hohen Druck versorgt wird, bietet das Versorgungsgerät mehrere verschiedene Anschlüsse in Form von Bohrungen 44 - 46. Beispielsweise kann die Bohrung 46 für den Anschluss einer Fluid Rückführung einer starren Sonde ausgelegt sein. Dieser Anschluss kann dann geräteseitig mit hohem Druck beaufschlagt werden. Dahingehend kann beispielweise die Bohrung 45 für den Anschluss einer flexiblen Sonde vorgesehen sein und kann geräteseitig nicht mit einem hohem Druck beaufschlagt werden. Bei einem auf diese Art und Weise ausgebildeten Instrumentenstecker 11 ist der Fluidstecker 28, der mit der Bohrung 44 in Wirkverbindung steht, als Fluid Versorgungsanschluss ausgebildet und wird sowohl von einer starren Sonde wie auch einer flexiblen Sonde verwendet. Dahingehend wird der Fluidstecker 30 zur Rückführung des Fluids einer starren Sonde und der Fluidstecker 29 zur Rückführung des Fluids einer flexiblen Sonde verwendet.

Bei dem Ausführungsbeispiel des Instrumentensteckers 11, dargestellt in Figur 2 und 6, handelt es sich um eine Basisausführung eines solchen. Wird ein Instrumentenstecker 11 beispielsweise für die Verwendung einer starren Sonde konfiguriert, entfällt beispielsweise die Fluidleitung 27 und der Fluidstecker 30. Dafür weist der konfigurierte Instrumentenstecker 11 ein Verschlussmittel an der Stelle, an der ansonsten der Fluidstecker 30 angebracht ist, auf, um das Gehäuse 17 zu verschließen. Somit ist es möglich, den Innenraum 24 des Gehäuses 17 mit der Vergussmasse 41 zu füllen. Entfällt die Fluidleitung 26 und der Fluidstecker 29 bei einer Konfiguration für eine flexible Sonde, wird anstelle des Fluidsteckers 29 ein Verschlussmittel verwendet, um das Ausgießen des Instrumentensteckers 11 zu ermöglichen.

Das erfindungsgemäße Instrument weist einen Instrumentenstecker 11 auf, dessen Fluidstecker 28 bis 30 an einer vorzugsweise ebenen Stirnseite 20 angeordnet sind. Vorzugsweise ist an der gleichen Stirnseite 20 eine zur Druckentlastung dienende Entlüftungsbohrung 38 angeordnet, über die in dem Instrument 14 gegebenenfalls und insbesondere im Fehlerfalle anfallende Leckfluide, insbesondere Leckgase, nach außen abgeführt werden können. Dieses Konzept ermöglicht es optional, eine Abdeckkappe 43 vorzusehen, die an die Stirnseite 20 angesetzt wird. So kann das Instrument 10 einschließlich des Instrumentensteckers 11 sterilisiert werden.

### Bezugszeichenliste:

- 10: Instrument
- 11: Instrumentenstecker
- 12: Instrumentenkopf
- 13: Werkzeug
- 14: Versorgungsleitung
- 15: Stecker
- 16: Leitung
- 17: Gehäuse
- 18: untere Gehäuseschale
- 19: obere Gehäuseschale
- 20: Stirnseite
- 21: Rückseite
- 22: Fassung
- 23: Mantel
- 24: Innenraum
- 25 - 27: Fluidleitungen
- 28 - 30: Fluidstecker
- 31 - 33: Kanal
- 34, 35: Dichtungen
- 36: Radialbohrung
- 37: Entlüftungsschlauch
- 38: Entlüftungsbohrung, Entlüftungsöffnung
- 39: Fortsatz
- 40: Innenraum
- 41: Vergussmasse
- 42: Gehäusefuge
- 43: Abdeckkappe
- 44 - 46: Bohrung
- 47: Stirnseite
- 48: Dichtungsmittel
- 49: Dichtung
- 50: Zapfen
- 51: Anwendungsteil
- 52: Fluidleitung

## Patentansprüche

1. Medizinisches Instrument (10):
mit einem Instrumentenkopf (12), der zur Anwendung an einem Patienten eingerichtet ist,
mit einer Versorgungsleitung (14), die mindestens zwei Fluidleitungen (25, 26) für ein gasförmiges, flüssiges oder superkritisches Fluid und mindestens einen Mantel (23) aufweist,
wobei die Fluidleitungen
(25, 26) sich durch den Innenraum (24) des Mantels (23) erstrecken und von denen eine Fluid zu dem Instrumentenkopf (12) hin und die andere Fluid von dem Instrumentenkopf (12) weg leitet,
mit einem Instrumentenstecker (11), der eine Stirnseite (20) und einen vollständig mit einer Vergussmasse (41) gefüllten Innenraum (40) aufweist,
mit mindestens zwei Fluidsteckern, (28,29), die mit den Fluidleitungen (25,26) in Fluidverbindung stehen und die an der Stirnseite (20) angeordnet sind,
und
mit wenigstens einer zur Druckentlastung dienenden Entlüftungsöffnung (38), die über einen Entlüftungsschlauch (37) oder ein entsprechendes Rohr mit dem Innenraum (24) des Mantels (23) zur Abfuhr anfallender Leckfluide in Verbindung steht und über die der Innenraum (24) mit der Atmosphäre in Verbindung steht, wobei die Entlüftungsöffnung an der Stirnseite (20) des Fluidsteckers (11) angeordnet ist,
wobei die Vergussmasse die Fluidleitungen (25, 26) und den Entlüftungsschlauch (37) umhüllt.

2. Instrument nach einem der vorstehenden Ansprüche, **dadurch gekennzeichnet, dass** das Fluid in Gebrauch unter einem Druck steht, der höher ist als der Umgebungsdruck.

3. Instrument nach Anspruch 1, **dadurch gekennzeichnet, dass** an der Stirnseite (20) die wenigstens zwei Fluidstecker (28, 29) zueinander parallel sowie zu der Stirnseite (20) rechtwinklig ausgerichtet sind.

4. Instrument nach Anspruch 3, **dadurch gekennzeichnet, dass** die Fluidstecker (28, 29) untereinander gleich ausgebildet sind.

5. Instrument nach Anspruch 3, **dadurch gekennzeichnet, dass** die Entlüftungsöffnung (38) zwischen den beiden Fluidsteckern (28, 29) angeordnet ist.

6. Instrument nach einem der vorstehenden Ansprüche, **dadurch gekennzeichnet, dass** die Versorgungsleitung (14) flexibel ausgebildet ist.

7. Instrument nach einem der vorstehenden Ansprüche, **dadurch gekennzeichnet, dass** der Instrumentenstecker (11) als Flachstecker ausgebildet ist, der von jeweils im rechten Winkel zu der Stirnseite (20) orientierten Flachseiten begrenzt ist.

8. Instrument nach einem der vorstehenden Ansprüche, **dadurch gekennzeichnet, dass** der Instrumentenstecker (11) zwei Gehäuseschalen (18, 19) umfasst, die an einer Fuge (42) aneinander anschließen.

9. Instrument nach Anspruch einem der vorstehenden Ansprüche, **dadurch gekennzeichnet, dass** die Stirnseite (20) fugenlos ausgebildet ist.

10. Instrument nach einem der vorstehenden Ansprüche, **dadurch gekennzeichnet, dass** der Fluidstecker (28) rohrförmig, endseitig geschlossen ausgebildet ist und einen inneren Kanal (31) aufweist, der mit einer Radialbohrung (36), kommuniziert.

11. Instrument nach Anspruch 10, **dadurch gekennzeichnet, dass** der Fluidstecker (28) zu beiden Seiten der Radialbohrung (36) Dichtungen (34, 35) aufweist.

12. Instrument nach einem der vorstehenden Ansprüche, **dadurch gekennzeichnet, dass** dem Instrumentenstecker (11) eine Abdeckkappe (43) zugeordnet ist, die an die Stirnfläche (20) anschließt.

13. Instrument nach einem der vorstehenden Ansprüche, **dadurch gekennzeichnet, dass** die Abdeckkappe (43) zum Verschluss der mindestens zwei Fluidstecker (28,29) und der Entlüftungsöffnung (38) eingerichtet ist.

## Claims

1. Medical instrument (10),
with an instrument head (12) designed for use on a patient,
with a supply line (14) which comprises at least two fluid lines (25, 26) for a gaseous, liquid or supercritical fluid, and at least one casing (23),
wherein the fluid lines (25, 26) extend through the interior (24) of the casing (23) and of which one conducts a fluid to the instrument head (30) and the other conducts a fluid away from the instrument head (12),
with an instrument connector (11) which has an end face (20) and an interior (40) totally filled with a casting compound (41),
with at least two fluid connectors (28, 29) which are in fluidic communication with the fluid lines (25, 26) and which are arranged on the end face (20),
with at least one venting opening (38) serving for pressure relief, which is connected via a venting hose (37) or a corresponding pipe to the interior (24) of the casing (23) to discharge any leakage fluid occurring, and via which the interior (24) is connected to atmosphere, wherein the venting opening is arranged on the end face (20) of the fluid connector (11),
wherein the casting compound envelopes the fluid lines (25, 26) and the venting hose (37).

2. Instrument according to any of the preceding claims, **characterised in that** in use, the fluid is under a pressure which is higher than the ambient pressure.

3. Instrument according to claim 1, **characterised in that** on the end face (20), the at least two fluid connectors (28, 29) are oriented parallel to each other and perpendicular to the end face (20).

4. Instrument according to claim 3, **characterised in that** the fluid connectors (28, 29) are configured identically.

5. Instrument according to claim 3, **characterised in that** the venting opening (38) is arranged between the two fluid connectors (28, 29).

6. Instrument according to any of the preceding claims, **characterised in that** the supply line (14) is configured flexibly.

7. Instrument according to any of the preceding claims, **characterised in that** the instrument connector (11) is configured as a flat connector delimited by flat sides which are each oriented perpendicularly to the end face (20).

8. Instrument according to any of the preceding claims, **characterised in that** the instrument connector (11) comprises two housing shells (18, 19) which adjoin each other at a joint (42).

9. Instrument according to any of the preceding claims, **characterised in that** the end face (20) is configured jointlessly.

10. Instrument according to any of the preceding claims, **characterised in that** the fluid connector (28) is configured tubular and closed at the end, and has an inner channel (31) which communicates with a radial bore (36).

11. Instrument according to claim 10, **characterised in that** the fluid connector (28) has seals (34, 35) on both sides of the radial bore (36).

12. Instrument according to any of the preceding claims, **characterised in that** a cover cap (43) which adjoins the end face (20) is assigned to the instrument connector (11).

13. Instrument according to any of the preceding claims, **characterised in that** the cover cap (43) is configured to seal the at least two fluid connectors (28, 29) and the venting opening (38).

## Revendications

1. Instrument médical (10) :
comprenant une tête d'instrument (12) qui est conçue pour une utilisation sur un patient,
comprenant une conduite d'alimentation (14) qui présente au moins deux conduites de fluide (25, 26) pour un fluide gazeux, liquide ou supercritique, et au moins une enveloppe (23), sachant que les conduites de fluide (25, 26) s'étendent à travers l'espace intérieur (24) de l'enveloppe (23) et l'une d'elles amène du fluide à la tête d'instrument (12) et l'autre évacue le fluide de la tête d'instrument (12),
comprenant un connecteur d'instrument (11) qui présente une face frontale (20) et un espace intérieur (40) rempli entièrement avec une masse de remplissage (41),
comprenant au moins deux connecteurs de fluide (28, 29) qui sont en communication de fluide avec les conduites de fluide (25, 26) et qui sont disposés sur la face frontale (20), et
comprenant au moins un orifice de ventilation (38) servant à l'élimination de pression, qui est relié à l'espace intérieur (24) de l'enveloppe (23) par l'intermédiaire d'un tuyau de ventilation (37) ou d'un tube correspondant, en vue de l'évacuation de fuites de fluides qui se produisent, et par l'intermédiaire duquel l'espace intérieur (24) communique avec l'atmosphère, l'orifice de ventilation étant disposé sur la face frontale (20) du connecteur de fluide (11),
la masse de remplissage entourant les conduites de fluide (25, 26) et le tuyau de ventilation (37).

2. Instrument selon l'une des revendications précédentes, **caractérisé en ce que** lors de l'utilisation, le fluide est soumis à une pression qui est supérieure à la pression ambiante.

3. Instrument selon la revendication 1, **caractérisé en ce que** sur la face frontale (20), les connecteurs de fluide (28, 29), au nombre d'au moins deux, sont orientés parallèlement les uns par rapport aux autres et perpendiculairement à la face frontale (20).

4. Instrument selon la revendication 3, **caractérisé en ce que** les connecteurs de fluide (28, 29) sont réalisés de façon identique entre eux.

5. Instrument selon la revendication 3, **caractérisé en ce que** l'orifice de ventilation (38) est disposé entre les deux connecteurs de fluide (28, 29).

6. Instrument selon l'une des revendications précédentes, **caractérisé en ce que** la conduite d'alimentation (14) est réalisée sous une forme souple.

7. Instrument selon l'une des revendications précédentes, **caractérisé en ce que** le connecteur d'instrument (11) est réalisé comme connecteur plat qui est délimité par des côtés plats orientés respectivement à angle droit par rapport à la face frontale (20).

8. Instrument selon l'une des revendications précédentes, **caractérisé en ce que** le connecteur d'instrument (11) comporte deux coques de boîtier (18, 19) qui se raccordent l'une à l'autre par un joint (42).

9. Instrument selon l'une des revendications précédentes, **caractérisé en ce que** la face frontale (20) est réalisée sans joints.

10. Instrument selon l'une des revendications précédentes, **caractérisé en ce que** le connecteur de fluide (28) est réalisé sous une forme tubulaire fermée aux extrémités et présente un canal interne (31) qui communique avec un trou radial (36).

11. Instrument selon la revendication 10, **caractérisé en ce que** le connecteur de fluide (28) présente des joints d'étanchéité (34, 35) de part et d'autre du trou radial (36).

12. Instrument selon l'une des revendications précédentes, **caractérisé en ce qu'**une coiffe de recouvrement (43), qui se raccorde à la face frontale (20), est associée au connecteur d'instrument (11).

13. Instrument selon l'une des revendications précédentes, **caractérisé en ce que** la coiffe de recouvrement (43) est conçue pour l'obturation des connecteurs de fluide (28, 29), au nombre d'au moins deux, et de l'orifice de ventilation (38).
